# EUROPEAN PATENT APPLICATION

(11) **EP 3 480 223 A1**
(43) Date of publication of application: **08.05.2019**
(21) Application number: 18204009.7
(22) Date of filing: 01.11.2018
(51) Int. Cl.: C08F 212/32, C08F 212/08, C08F 220/18, C08F 226/06, C09D 125/02

(54) **LOW TEMPERATURE CURABLE ADDITION POLYMERS FROM VINYL ARYLCYCLOBUTENE-CONTAINING MONOMERS AND METHODS FOR MAKING THE SAME**

(30) Priority: 02.11.2017 US 201715801784
(71) Applicant: Rohm and Haas Electronic Materials LLC, Marlborough, MA 01752 (US)
(72) Inventor: HAYES, Colin, Marlborough, MA 01752 (US); AOUDE, Tina, Marlborough, MA 01752 (US); BARR, Robert K., Marlborough, MA 01752 (US); FLEMING, David, Marlborough, MA 01752 (US); GALLAGHER, Michael K., Marlborough, MA 01752 (US); PROKOPOWICZ, Gregory D., Marlborough, MA 01752 (US); RIENER, Michelle, Marlborough, MA 01752 (US)
(74) Representative: Houghton, Mark Phillip

(57) **Abstract**

The present invention provides a low temperature polymerizing and curing polymer composition comprising, in copolymerized form, one or more addition polymerizable arylcyclobutene monomers A having as a cyclobutene ring substituent one or more groups chosen from alkyl; heteroatom containing alkyl; aryl; heteroatom containing aryl; or heteroatom containing aryloxy, one or more aromatic addition polymerizable second monomers, and one or more other addition polymerizable monomers chosen from an addition polymerizable nitrogen heterocycle containing third monomer, an addition polymerizable fourth monomer, or, preferably, both of the one or more third monomers and the one or more fourth monomers. The polymer compositions find use in making films or coatings for use in electronics applications.

## Description

The present invention relates generally to the field of polymer materials, and, more particularly, to polymers of, in copolymerized form, a monomer mixture of one or more addition polymerizable arylcyclobutene-containing monomers A having one or more groups chosen from alkyl, heteroatom containing alkyl, alkoxy, aryl, aryloxy, heteroatom containing aryl or heteroatom containing aryloxy, one or more aromatic addition polymerizable second monomers and one or more other addition polymerizable monomers chosen from an addition polymerizable nitrogen heterocycle containing third monomer, an addition polymerizable fourth monomer or both.

Because of their low dielectric constant and low dielectric loss, arylcyclobutene-containing polymers are used as dielectric materials in a variety of electronic applications, such as microelectronic packaging and interconnect applications. While arylcyclobutene-containing monomers having a cyclobutene ring substituents have been known to ring open at a temperature below 200 °C, the cure rate is insufficient at these temperatures to enable ready production of a polymer having an acceptably high molecular weight (Mw (GPC) of 6,000 or higher) suitable for electronics applications. Further, at such cure temperatures known addition polymerizable crosslinkers, such as diacrylates or multi-functional acrylates, tend to self-polymerize before the arylcyclobutene ring opens.

U.S. patent publication no. 2015/0210793 A1 to Park et al. discloses crosslinkable polymers from addition polymerizable arylcyclobutene-containing monomers and second monomers, such as a (meth)acrylate, wherein the compositions enable the provision of polymers that crosslink at temperatures lower than the ring opening temperature of conventional arylcyclobutene-containing polymers. However, Park et al. disclose compositions that prove to lack sufficient amounts of arylcyclobutene-containing monomers to insure their usefulness in electronics applications.

The present inventors have sought to solve the problem of providing a dielectric material that cures at a lower temperature while retaining the low dielectric constant and low dielectric loss of poly (benzo)cyclobutenes.

### STATEMENT OF THE INVENTION

In accordance with a polymer composition of the first aspect of the present invention, polymer compositions comprise at least one polymer of, in copolymerized form, a monomer mixture of one or more addition polymerizable arylcyclobutene-containing monomers A having as a cyclobutene ring substituent one or more groups chosen from alkyl; heteroatom containing alkyl, such as alkoxy or alkylthio; aryl; heteroatom containing aryl, such as aryloxy or thioaryl; or heteroatom containing aryloxy, one or more aromatic addition polymerizable second monomers, and one or more other addition polymerizable monomers chosen from an addition polymerizable nitrogen R₁ heterocycle containing third monomer, an addition polymerizable fourth monomer, or, preferably, both of the one or more third monomers and the one or more fourth monomers.

In accordance with the polymer composition of the first aspect of the present invention, the polymer compositions comprise at least one polymer of, in copolymerized form, a monomer mixture of one or more addition polymerizable arylcyclobutene-containing monomers A having as a cyclobutene ring substituent one or more groups chosen from alkyl and aryl and the polymer compositions further comprise an addition polymerizable crosslinker monomer containing two or more allyl groups, such as triallyl isocyanurate, a diallyl glycol, a diallyl polyether diol, a diallyl polyester diol, preferably, a diallyl aromatic polyester diol, or triallyl benzene.

In accordance with the polymer composition of the first aspect of the present invention, the polymer composition comprises, in copolymerized form, a monomer mixture of the one or more arylcyclobutene-containing monomers A, the one or more aromatic addition polymerizable second monomers, such as styrene and the one or more other addition polymerizable monomers chose from the third monomer, the fourth monomer or both; wherein monomer A has Structure A-1 or a Structure A-2: 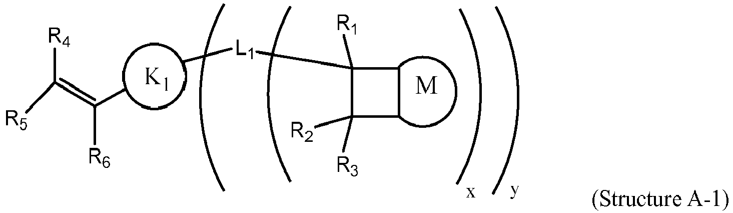 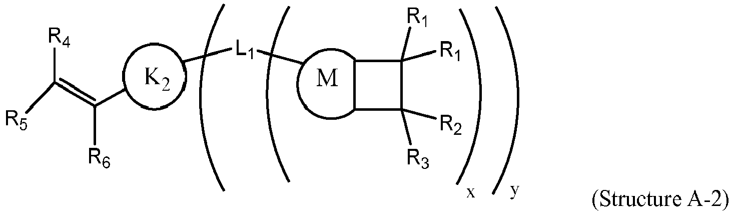
wherein, K₁ is a divalent heteroatom containing hydrocarbon group having from 1 to 36 carbon atoms, such as an alkylthio or alkoxy group, for example, a C₁ to C₆ alkoxy group, or such as an an aryloxy group or an arylthio group, for example, a C₁ to C₆ alkyl substituted or unsubstituted divalent O-heteroaryl group or S-heteroaryl group having from 5 to 36 carbon atoms; a divalent hydrocarbon group having from 5 to 36 carbon atoms, such as an alkyl, alkylene, aryl or alkylaryl group; a cyano group; a carbonyl group; an ester group (-COO-); a carboxyl group (-OOC-); or a divalent heteroatom group, such as an ether group (-O-); or a thioether group (-S-);
K₂ is a covalent bond or a divalent group chosen from a C₁ to C₆ alkyl substituted or unsubstituted divalent aryl group, a C₁ to C₆ alkyl substituted or unsubstituted divalent hydrocarbon group, or a divalent C₁ to C₃₀ alkylene group, such as a C₁ to C₆ alkylene group; a divalent heteroatom containing hydrocarbon group, such as a C₁ to C₆ alkyl substituted or unsubstituted divalent heteroaryl group, for example, an aryloxy group, an arylthio group, or a C₁ to C₆ alkyl substituted or unsubstituted divalent O-heteroaryl group or S-heteroaryl group having from 5 to 36 carbon atoms or such as a C₁ to C₆ alkyl substituted or unsubstituted divalent heteroalkyl group, for example, an alkoxy group, such as a C₁ to C₆ alkoxy group, or an alkylthio group; an ether group (-O-); or a thioether group (-S-), a carbonyl group; an ester group (-COO-); a carboxyl group (-OOC-) or a cyano group; or, preferably, K₂ is a covalent bond;
M is a divalent aromatic group chosen from a C₁ to C₆ alkyl substituted or unsubstituted aromatic radical group, or a C₁ to C₆ alkyl substituted or unsubstituted divalent heteroaromatic radical group;
L₁ is a covalent bond or Lᵢ is a linking group, such as a hydrocarbon linking group, having a valence of x +1, or, preferably, is a covalent bond; and, R₁ through R₆ are each independently selected from a monovalent group chosen from hydrogen, deuterium, halogen, hydroxyl a C₁ to C₆ alkyl group, a C₁ to C₆ alkoxy group, a C₁ to C₆ alkyl substituted hydrocarbon group, a heteroatom containing hydrocarbon group, a C₁ to C₆ alkyl substituted heterohydrocarbon group, a cyano group, an hydroxyl group, a monovalent aryl group, a C₁ to C₆ alkyl substituted aryl group, a heteroaryl group, or a C₁ to C₆ alkyl substituted heteroaryl group;
x and y are each independently an integer from 1 to 5, or, preferably, from 1 to 2, or, more preferably, 1, and wherein y is 1 when Li is a covalent bond;
wherein, when the one or more arylcyclobutene-containing monomers A has the structure A-1, each of R₁, R₂ and R₃ is a hydrogen; and,
further wherein, when the one or more arylcyclobutene-containing monomers A has the structure A-2, L₁ is a covalent bond or a hydrocarbon linking group; or, preferably, both, of L₁ and K₂ are a covalent bonds; and at least one of R₁, R₂ and R₃ is chosen from a C₁ to C₆ alkyl group; a C₁ to C₆ alkoxy group; a C₁ to C₆ alkyl substituted hydrocarbon group having from 5 to 36 carbon atoms; a heteroatom containing hydrocarbon group; a C₁ to C₆ alkyl substituted heterohydrocarbon group, such as thioalkyl; a cyano group; an aryl group; a C₁ to C₆ 6 alkyl substituted aryl group, a heteroaryl group, such as aryloxy or thioaryl; or a C₁ to C₆ alkyl substituted heteroaryl group; or, preferably, the at least one of R₁, R₂ and R₃ is a group chosen from a C₁ to C₆ alkyl group, a C₁ to C₆ alkoxy group, an aryl group, an aryloxy group, an hydroxyl group, a thioalkyl group, or a thioaryl group.

In accordance with the polymer composition of the first aspect of the present invention, the polymer compositions comprise at least one polymer of, in copolymerized form, a monomer mixture of one or more addition polymerizable arylcyclobutene-containing monomers A (i) having the Structure A-1 and L₁ and K together comprise an alkyl, an alkylene, an aryl or an arylene group, or (ii) having the Structure A-2 wherein one or both of L₁ and K are a covalent bond or together form a C₁ to C₆ alkylene radical, and at least one of R₁, R₂ and R₃ is chosen from a C₁ to C₆ alkyl group; a C₁ to C₆ alkyl substituted hydrocarbon group having from 5 to 36 carbon atoms; an aryl group; or a C₁ to C₆ alkyl substituted aryl group, the polymer compositions further comprise an addition polymerizable crosslinker monomer containing two or more allyl groups, such as triallyl isocyanurate, a diallyl glycol, a diallyl polyether diol, a diallyl polyester diol, preferably, a diallyl aromatic polyester diol, or triallyl benzene.

In accordance with the compositions of the first aspect of the present invention, the arylcyclobutene polymers of the present invention may comprise, in copolymerized form, one arylcyclobutene-containing monomer A or more than one arylcyclobutene-containing monomer A.

Preferably, in accordance with the compositions of the first aspect of the present invention, the arylcyclobutene-containing monomer A comprises an α-vinyl phenoxy benzocyclobutene, α-vinyl methoxy benzocyclobutene, α-vinyl phenyl BCB or an α-vinyl hydroxynaphthalene BCB monomer of structure A-1, a vinyl α-alkoxy benzocyclobutene, a vinyl α-phenoxy benzocyclobutene, or a vinyl α-C₁ to C₆ alkyl benzocyclobutene monomer of structure A-2, such as a vinyl α-methyl benzocyclobutene.

Preferably, in accordance with the compositions of the first aspect of the present invention, the polymer composition comprises a polymer of, in copolymerized form, a monomer mixture of the one or more addition polymerizable arylcyclobutene-containing monomers A, the one or more aromatic addition polymerizable second monomers chosen from styrene, α-methyl styrene, 8-myrcene, allyloxystyrene, allyl terminated polyarylene ethers or maleimide terminated polyarylene ethers; and the nitrogen heterocycle containing addition polymerizable third monomer.

Preferably, in accordance with the compositions of the first aspect of the present invention, the polymer composition comprises a polymer of, in copolymerized form,a monomer mixture of the one or more addition polymerizable arylcyclobutene-containing monomers A; the one or more aromatic addition polymerizable second monomers; and the one or more nitrogen heterocycle containing addition polymerizable third monomer chosen from a nitrogen heterocycle containing third monomer vinyl monomer, such as N-vinyl pyridine, N-vinyl imidazole or other vinyl pyridine isomers such as 2-vinyl pyridine, or, more preferably, two or more addition polymerizable group containing nitrogen heterocycle containing third monomers, such as N-vinyl pyridine and N-vinyl imidazole.

Preferably, in accordance with the compositions of the first aspect of the present invention, the polymer composition comprises a polymer of, in copolymerized form, a monomer mixture of the one or more addition polymerizable arylcyclobutene-containing monomers A, the one or more aromatic addition polymerizable second monomers, and the one or more other addition polymerizable monomers which is one or more addition polymerizable fourth monomers chosen from acrylates or methacrylates, such as cyclohexyl methacrylate, n-butyl methacrylate, cyclohexyl acrylate and fatty (meth)acrylates; maleimides and bis-maleimides; cyclic anhydrides, such as maleic anhydride, 3-methylenedihydrofuran-2,5-dione or itaconic anhydride; (meth)acrylates for improving adhesion promotion, such as 2-acryloyloxyethylacid phosphate, trimethoxysilylpropyl methacrylate, 2[2-hydroxy-5-[2-(methacryloyloxy)ethyl]phenyl]-2H-benzotriazole,2-(methacryloyloxy)ethyl acetoacetate; allyl group containing monomers for adhesion promotion, such as allyl phosphonates, allyloxyethyl phosphate, or allyloxypropyl trimethoxysilane; linear and branched alkenes, such as hexene; cyclic olefins, such as cyclopentene, cyclohexene or cyclooctene; and fourth monomers containing a second dienophile or addition polymerizable group, such as benzocyclobutene (BCB) containing crosslinkers, for example, vinyl benzocyclobutene, or divinylsiloxyl bis-benzocyclobutene (DVS-BCB); allyl methacrylate; divinyl benzene; dienes, such as, butadiene, cyclopentadiene, β-myrcene, ocimene, cyclooctadiene, or tetraphenylcyclopentadienone; allyloxystyrene; vinyl, allyl, or maleimide terminated polyols, oligosiloxanes or polysiloxanes; or maleimide terminated polyimides; preferably, the fourth monomer comprises n-butyl acrylate, cyclohexyl acrylate, cyclohexyl methacrylate or C₈ to C₁₈ alkyl (meth)acrylates, such as fatty (meth)acrylates.

In accordance with the compositions of the first aspect of the present invention, for lower boiling monomers (below 150 °C), such as, C₁ to C₆ alkyl (meth)acrylates, hexene, cyclopentene, cyclohexene, butadiene, and cyclopentadiene, a pressurized reactor can be used to prevent loss through evaporation of the unsaturated compound prior to reaction with the arylcyclobutene-containing monomers A.

In accordance with the first aspect of the present invention, the polymer composition may comprise, in copolymerized form, a monomer mixture of from 10 to 90 wt.% or, preferably, from 15 to 70 wt.% of the one or more arylcyclobutene-containing monomers A; from 5 to 70 wt.% or, preferably, from 25 to 55 wt.% of the one or more aromatic addition polymerizable second monomer; from 5 to 40 wt.%, or, preferably, from 10 to 40 wt.%, or, more preferably, from 15 to 35 wt.% of the one or more other addition polymerizable monomers which is the one or more nitrogen heterocycle containing addition polymerizable third monomers, the one or more addition polymerizable fourth monomers, such as a (meth)acrylate monomer, or both, all weights based on the total solids weight of monomers used to make the copolymer with all monomer wt.%s adding to 100%.

Preferably, in accordance with the first aspect of the present invention, the polymer composition may comprise, in copolymerized form, a monomer mixture of from 10 to 40 wt.% or, more preferably, from 15 to 35 wt.% of the one or more other addition polymerizable monomers which is nitrogen heterocycle containing addition polymerizable third monomer plus the one or more addition polymerizable fourth monomers, such as a (meth)acrylate monomer, based on the total solids weight of the monomers used to make the copolymer with all monomer wt.%s adding to 100%.

In accordance with the compositions of the first aspect of the present invention, the polymer composition may further comprise a separate crosslinker, such as a bis-BCB monomer, for example, divinylsiloxy bis-benzocyclobutene (DVS-BCB), or a monomer containing two or more dienophile groups, such as divinyl benzene, 9,9-bis(4-vinylbenzyl)-9H-fluorene, or, preferably, a monomer containing two or more allyl groups; a curing agent, such as an initiator, a photoinitiator or other photoactive compound; a catalyst, or more than one of these. The polymer composition can be crosslinked via thermally induced or light induced initiation in the presence of the curing agent and a monomer containing two or more dienophile groups which are addition polymerizable, or by ring opening of the arylcyclobutene groups on the polymer in the presence of any monomer containing two or more dienophile groups.

In accordance with the compositions of the first aspect of the present invention, the polymer composition may comprise, in copolymerized form, the monomer mixture of the one or more addition polymerizable arylcyclobutene monomers A, the one or more aromatic addition polymerizable second monomer, as the one or more other addition polymerizable monomers, the one or more addition polymerizable nitrogen heterocycle containing third monomers, and, if desired, the one or more addition polymerizable fourth monomers, wherein the composition further comprises a separate crosslinker chosen from a crosslinking monomer, such as monomer containing a second addition polymerizable or dienophile group, and wherein the composition can be crosslinked via thermally induced or light induced initiation in the presence of a curing agent, such as one chosen from a thermal initiator, such as an oil soluble initiator, preferably, dimethyl 2,2'-azobis(2-methylpropionate); a photoactive compound; or a photoinitiator.

In accordance with the first aspect of the present invention, the polymer composition further comprises a separate crosslinker having two or more arylcyclobutene-containing groups, such as a bis-BCB monomer, for example, divinylsiloxy bis-benzocyclobutene (DVS-BCB).

Preferably, in accordance with the compositions of the first aspect of the present invention, the compositions further comprise one or more addition polymerizable crosslinker monomers having a formula weight of 500 or less and containing two or more, or, more preferably, three or more addition polymerizable groups, such as allyl or (meth)acrylate groups, or, more preferably, propoxylated trimethylolpropane triacrylate, tricyclodecanedimethanol diacrylate, triallyl isocyanurate (TAIC), or bis maleimides, such as the bis-maleimide of 2,2,4-trimethyl hexane (BMI-TMH), with the proviso that when the arylcyclobutene-containing monomers A comprise alkyl, aryl or alkyl aryl substituents on the arylcyclobutene ring or linking the arylcyclobutene ring to an addition polymerizable group the addition polymerizable crosslinker monomer comprises two or more allyl groups.

In accordance with the compositions of the first aspect of the present invention, the molar ratio of addition polymerizable groups in the one or more crosslinker monomers to the number molar equivalents of arylcyclobutene groups from the monomers A may range from 0.25:1 to 2.0:1 , or, preferably, 0.9:1 to 1.5:1.

In accordance with the first aspect of the present invention, the polymer composition comprises from 10 to 80 wt.%, or, preferably, from 20 to 50 wt.% of polymer solids, based on the total weight of the polymer and the remainder of an organic solvent, such as a polar protic solvent, such as an aliphatic ketone, like 2-butanone; an alkyl glycol ether, such as propylene glycol methyl ether; or a (cyclo)alkanol; or a polar aprotic solvent, such as an alkyl ester, amide or sultone.

In accordance with a second aspect of the present invention, a thin film or coating on a substrate comprises the polymer composition of the first aspect of the present invention of, in copolymerized form, one or more addition polymerizable arylcyclobutene-containing monomer A having as a cyclobutene ring substituent one or more alkyl, alkoxy, aryl, aryloxy, heteroatom containing alkyl, such as alkylthio, heteroatom containing aryl, such as aryloxy or heteroatom containing aryloxy, cyano or hydroxyl group, the one or more aromatic addition polymerizable second monomers, such as styrene, and the one or more other addition polymerizable monomers chosen from one or more nitrogen heterocycle containing addition polymerizable third monomers, such as a vinyl pyridine or a vinyl imidazole, one or more addition polymerizable fourth monomers, or, preferably, both.

In accordance with the thin film or coating of the second aspect of the present invention, wherein the one or more monomers A has the above Structure A-1 or A-2.

In accordance with the thin film or coating of the second aspect of the present invention, the polymer composition comprises, in copolymerized form, the monomer mixture of one or more arylcyclobutene-containing monomer A of the first aspect of the present invention, one or more aromatic addition polymerizable second monomers of the first aspect of the present invention, and the one or more other addition polymerizable monomers of the first aspect of the present invention.

In accordance with the thin film or coating of the second aspect of the presentinvention, the polymer composition may comprise, in copolymerized form, from 10 to 90 wt.% or, preferably, from 15 to 70 wt.% of the one or more arylcyclobutene-containing monomers A; from 5 to 70 wt.% or, preferably, from 20 to 55 wt.% of the one or more aromatic addition polymerizable second monomer; from 5 to 40 wt.% or, preferably, from 10 to 30 wt.% of the one or more other addition polymerizable monomers which is chosen from the one or more nitrogen heterocycle containing addition polymerizable third monomers, the one or more addition polymerizable fourth monomers, such as a (meth)acrylate monomer, or mixtures thereof, all weights based on the total solids weight of monomers used to make the copolymer with all monomer wt.%s adding to 100%.

Preferably, in accordance with the second aspect of the present invention, the polymer composition may comprise, in copolymerized form, from 10 to 40 wt.%, or, preferably, from 10 to 30 wt.% of the one or more nitrogen heterocycle containing addition polymerizable third monomer plus the one or more addition polymerizable group containing fourth monomers, based on the total solids weight of the monomers used to make the copolymer with all monomer wt.%s adding to 100%.

In accordance with the thin film or coating of the second aspect of the present invention, the coating may further comprise, in reacted or copolymerized form, a condensation crosslinker, such as a diamine; a separate crosslinker monomer having two or more arylcyclobutene-containing groups, such as a bis-BCB monomer, for example, divinylsiloxy bis-benzocyclobutene (DVS-BCB); or a crosslinker monomer containing two or more dienophile or addition polymerizable groups, such as divinyl benzene or 9,9-bis(4-vinylbenzyl)-9H-fluorene, or, preferably, a monomer containing two or more allyl groups; a curing agent, such as an initiator, one chosen from a thermal initiator, such as an oil soluble initiator, preferably, dimethyl 2,2'-azobis(2-methylpropionate); a photoinitiator or other photoactive compound; a catalyst, or more than one of these.

Preferably, in accordance with the thin film or coating of the second aspect of the present invention, the coating further comprises, in copolymerized form, one or more crosslinker monomers containing two or more, or, more preferably, three or more addition polymerizable groups, such as allyl or (meth)acrylate groups, or, more preferably, propoxylated trimethylolpropane triacrylate, tricyclodecanedimethanol diacrylate, triallyl isocyanurate (TAIC), or bis-maleimides, such as the bis-maleimide of 2,2,4-trimethyl hexane (BMI-TMH).

In accordance with the thin film or coating of the second aspect of the present invention, the coating comprises at least one polymer of, in copolymerized form, a monomer mixture of one or more addition polymerizable arylcyclobutene-containing monomers A (i) having the Structure A-1 and L₁ and K together comprise a divalent alkyl, an alkylene, an aryl or an arylene group or (ii) having the Structure A-2 wherein one or both of L₁ and K are a covalent bond or together form a C₁ to C₆ divalent alkylene group, and at least one of R₁, R₂ and R₃ is chosen from a C₁ to C₆ alkyl group; a C₁ to C₆ alkyl substituted hydrocarbon group having from 5 to 36 carbon atoms; an aryl group; or a C₁ to C₆ alkyl substituted aryl group, and, further, comprises an addition polymerizable crosslinker monomer containing two or more allyl groups, such as triallyl isocyanurate, a diallyl glycol, a diallyl polyether diol, a diallyl polyester diol, preferably, a diallyl aromatic polyester diol, or triallyl benzene.

In accordance with the thin film or coating of the second aspect of the present invention, the molar ratio of addition polymerizable groups in the one or more crosslinker monomers to the number molar equivalents of arylcyclobutene groups from the monomers A may range from 0.25:1 to 2.0:1, or, preferably, 0.9:1 to 1.5:1.

In accordance with a third aspect of the present invention, an electronic device contains a dielectric layer comprising the thin film or coating of the second aspect of the present invention on a substrate of the present invention, such as a polymer substrate, for example, polyethylene terephthalate or a polyimide.

In accordance with a fourth aspect of the present invention, a method of making a polymer composition comprises providing an organic solvent such as a polar protic solvent or a polar aprotic solvent, and a monomer mixture of one or more one or more addition polymerizable arylcyclobutene-containing monomers A having as a cyclobutene ring substituent one or more alkyl, alkoxy, aryl, aryloxy or other heteroaryl or hetroalkyl group, preferably, having the above Structure A-1 or A-2; the one or more aromatic addition polymerizable second monomers of the first aspect of the present invention, such as styrene, α-methyl styrene, β-myrcene, allyloxystyrene, allyl terminated polyarylene ethers or maleimide terminated polyarylene ethers; and the one or more other addition polymerizable monomers of the first aspect of the present invention chosen from nitrogen heterocycle containing addition polymerizable third monomers, such as a vinyl pyridine or a vinyl imidazole, preferably, two or more such monomers; addition polymerizable fourth monomers of the first aspect of the present invention; or both; and an initiator, such as an oil soluble thermal initiator or a photo-initiator, and polymerizing the monomer mixture. Where a thermal initiator is used, polymerizing further comprises heating the monomer mixture to from room temperature or ambient temperature to less than 140 °C or, preferably, from 60 to 125 °C, or more preferably, less than 110 °C.

In accordance with the methods of the fourth aspect of the present invention, the polymerizing can be followed by crosslinking or curing the resulting polymer in the presence of, respectively, a crosslinker monomer containing two or more dienophile groups or, preferably, two or more addition polymerizable groups, a curing agent or both at from ambient temperature to 140 °C for addition crosslinking in the presence of an initiator and at from 160 to 220 °C for ring opening curing.

In accordance with the methods of the fourth aspect of the present invention, the one or more monomers A is a monomer A as in the first aspect of the present invention, or, preferably has the above Structure A-1 or A-2 and wherein the one or more monomers A has the above Structure A-1 or A-2.

The proportions of organic solvent in accordance with the fourth aspect of the present invention are such as to form a polymer composition comprising from 10 to 80 wt.%, or, preferably, from 20 to 50 wt.% of polymer solids, based on the total weight of the polymer and aqueous alkali or organic solvent.

In accordance with the methods of the fourth aspect of the present invention, the monomer mixture or preferred monomer mixture is any that are useful or preferred in the first aspect of the present invention.

In accordance with a fifth aspect of the present invention, methods of making thin films or coatings on a substrate comprise depositing and evaporating or coating on a substrate the polymer composition of the first aspect of the present invention. The polymer compositions can comprise one or more organic solvents. The polymer compositions preferably, further comprise a curing agent chosen from a thermal initiator or a light initiator, a photoactive compound, if desired, with a crosslinker monomer containing two or more addition polymerizable groups or a monomer containing two or more dienophile groups, or a condensation crosslinker, such as a diamine or protected diamine.

In accordance with the methods of the fifth aspect of the present invention, the polymer can be heated in the presence of a curing agent, such as an initiator or a photoinitiator, and, if desired, an addition polymerizable crosslinker, at a temperature of from room temperature or ambient temperature to less than 140°C or, preferably, from 60 to 125 °C, or more preferably, less than 110 °C.

In accordance with the methods of the fifth aspect of the present invention, the methods can further comprise ring opening curing the film or coating at a temperature of from 160 to 220 °C or, preferably, from 180 to 210 °C.

The crosslinker or curing agent in accordance with the methods of the fifth aspect of (using the polymer composition of) the present invention can be any one or more of the crosslinkers or curing agents disclosed for use in the polymer composition of the first aspect of the present invention, disclosed above.

In accordance with a sixth aspect of the present invention, methods of using the polymer compositions of the first aspect of the present invention comprise depositing and evaporating or coating on a circuit substrate the polymer composition of the first aspect of the present invention to form a polymer layer, preferably with heating to a temperature of from 70 to 140 °C to effect a soft bake; photo-curing the polymer layer to obtain a photo-cured layer; and thermally curing the photo-cured layer, such as by ring opening at a temperature of from 160 to 220 °C to form an insulation layer.

Unless otherwise indicated, conditions of temperature and pressure are ambient or room temperature (RT) and standard pressure. All ranges recited are inclusive and combinable.

Unless otherwise indicated, any term containing parentheses refers, alternatively, to the whole term as if no parentheses were present and the term without them, and combinations of each alternative. Thus, the term "(meth)acrylate" refers to an acrylate, a methacrylate, or mixtures thereof.

As used herein, all amounts are percent by weight and all ratios are molar ratios, unless otherwise noted.

All numerical ranges are inclusive of the endpoints and combinable in any order, except where it is clear that such numerical ranges are constrained to add up to 100%.

As used herein, the articles "a", "an" and "the" refer to the singular and the plural. As used herein, the term "alkyl" includes linear, branched and cyclic alkyl. Likewise, "alkenyl" refers to linear, branched and cyclic alkenyl. " Aryl" refers to aromatic carbocycles and aromatic heterocycles.

As used herein, the term "aliphatic" refers to an open-chain carbon-containing moiety, such as alkyl, alkenyl and alkynyl moieties, which may be linear or branched. Also as used herein, the term "alicyclic" refers to a cyclic aliphatic moiety, such as cycloalkyl and cycloalkenyl. Such alicyclic moieties are non-aromatic, but may include one or more carbon-carbon double bonds. "Halo" refers to fluoro, chloro, bromo, and iodo. The term "(meth)acrylate" refers to both methacrylate and acrylate, and likewise the term (meth)acrylamide refers to both methacrylamide and acrylamide.

Unless the context clearly indicates otherwise, by "substituted" alkyl, alkenyl, or alkynyl is meant that one or more hydrogens on the alkyl, alkenyl, or alkynyl is replaced with one or more substituents chosen from halo, hydroxy, C₁₋₁₀ alkoxy, amino, mono- or di-C₁₋₁₀ hydrocarbyl substituted amino, C₅₋₂₀ aryl, and substituted C₅₋₂₀ aryl.

Unless the context clearly indicates otherwise, by "substituted" aryl is meant that one or more hydrogens on the aryl is replaced by one or more substituents chosen from halo, hydroxy, C₁₋₁₀ alkyl, C₂₋₁₀ alkenyl, C₂₋₁₀ alkynyl, C₁₋₁₀ alkoxy, amino, mono-or di-C₁₋₁₀ hydrocarbyl substituted amino, C₅₋₂₀ aryl, and substituted C₅₋₂₀ aryl. "Alkyl" refers to an alkane radical, and includes alkane diradicals (alkylene) and higher-radicals. Likewise, the terms "alkenyl", "alkynyl" and "aryl" refer to the corresponding mono-, di- or higher-radicals of an alkene, alkyne and arene, respectively.

As used herein, the term "addition polymerizable group" means any unsaturation functional group that polymerizes via addition polymerization, including vinyl, vinylidene or allyl groups and any such group having any alkyl, alkoxy, S, N, P, O or Si heteroatom containing hydrocarbon substituent or component, or any siloxy, cyano, aryl, alkylaryl, S, N, P, O or Si heteroatom containing aryl group, carbonyl, carboxyl(ate), aldehyde, diketo, hydroxyl, amine, imine, azo, phosphorus or sulfur containing group as a substituent or component.

As used herein, the term "aromatic organic residue" embraces an organic residue that has only aromatic character, such as phenyl, as well as an organic residue that contains a combination of aromatic and aliphatic moieties.

As used herein, the term "curing" is meant any process, such as addition crosslinking or condensation, that increases the molecular weight of a polymer material or composition through the use of the methods making or using the compositions in accordance with the present invention. "Curable" refers to any polymer material capable of being cured under certain conditions.

As used herein, the term "ASTM" refers to publications of ASTM International, West Conshohocken, PA.

As used herein, the term "complete cure" means that >98% of the residual heat of reaction of a given composition from an original cure profile has been removed from the composition when re-scanned by DSC, as compared to the known heat content of the composition. This is defined as the peak area integrated on the DSC trace, which corresponds to a heat content such as, for example, 200 Joules per gram (J/g). For a composition having a heat content of 200 J/g, a peak that had 100 J/g after a given thermal treatment (e.g. 200 °C for 1hr) would be considered 50% cured.

As used herein, the term "DSC" or "Differential Scanning Calorimetry" refers to a method of measuring polymer cure profiles or exotherms using a Q2000™ DSC instrument (TA Instruments, New Castle, DE). DSC was carried out using a sample of isolated uncured polymer (<5 mg) placed in a sealed Tzero™ Aluminum hermetic sample pan (TA instruments). The sample pan was then put in the DSC cell along with a control pan and the DSC was then heated from RT to 300 °C at a rate of 10 °C per minute. For B-staging an arylcyclobutene containing polymer, the amount of heat (J/g) liberated from a given sample between 200 and 300°C is defined to be the "residual degree of cure" or residual heat of reaction due to the arylcyclobutene ring opening reaction which has a peak exotherm of 258 °C.

As used herein, the term "formula weight" refers to the molecular weight of a representative formula depicting a given material.

As used herein, the term "hetero" or "heteroatom" when used referring to an organic group means an O, P, N, S or Si atom.

As used herein, the term "normal boiling point" refers to the boiling point of a given liquid in neat form at standard pressure.

As used herein, the term "oligomer" refers to relatively low molecular weight materials such as dimers, trimers, tetramers, pentamers, hexamers, and the like, including B-staged polymerized material, that are capable of further curing or polymerization.

As used herein, the term "solids" refers to any materials that remain a reaction product of the present invention; thus, solids include monomers and non-volatile additives that do not volatilize upon any of B-staging, polymerization and cure. Solids exclude water, ammonia and volatile solvents.

As used herein, the term "stoichiometry" of a reaction mixture refers to the ratio of molar equivalents of unreacted olefin groups or dienophile groups to unreacted arylcyclobutene groups in a given composition.

As used herein, unless otherwise indicated, the term "weight average molecular weight "or "Mw" means that value determined by gel permeation chromatography (GPC) of a polymer solution in tetrahydrofuran (THF) at room temperature using a Waters Alliance High Pressure Liquid Chromatogram (HPLC) (Waters, Milford, MA) equipped with an isocratic pump, an autosampler (Injection volume (100-150 µI) and a Series of 4 ShodexTM (8 mm x 30 cm) columns, each filled with a polystyrene divinyl benzene (PS/DVB) gel against a standard calibrated from polystyrene as standards. As used herein, "number average molecular weight "or "Mn" is measured in the same way as weight average molecular weight and represents the median molecular size in a given polymer composition. As used herein, the term "PDI" refers to the ratio of Mw/Mn.

As used herein, the term "wt.%" stands for weight percent.

As used throughout this specification, the following abbreviations shall have the following meanings, unless the context clearly indicates otherwise: °C = degree Celsius; min. = minutes; hr = hours; g = gram; L = liter; µm = micron = micrometer; nm = nanometer; mm = millimeter; mL = milliliter; MPa = megapascal; Mw = weight average molecular weight; Mn = number average molecular weight; and AMU = atomic mass unit. Unless otherwise noted, "wt.%" refers to percent by weight, based on the total weight of a referenced composition.

The inventors have found that compositions of polymers made from amounts of 10 to 90 wt.% of addition polymerizable arylcyclobutene monomers A having on the cyclobutene ring pendant groups, such as alkyl (methyl), alkoxy, thioalkyl, aryl, thioaryl or aryloxy (phenoxy) groups enable low temperature polymerization and cure without causing homopolymerization of the addition polymerizable comonomer. Such monomers A include, for example, vinyl methyl benzocyclobutene or vinyl phenoxy benzocyclobutene. The polymer compositions include copolymers of addition polymerizable arylcyclobutene-containing monomers A, in copolymerized form, with addition polymerizable aromatic second monomers and other addition polymerizable monomers, such as addition polymerizable nitrogen heterocycle containing monomers. More preferably, the compositions further comprise crosslinker monomers having two or more, e.g three reactive allyl groups. The crosslinker monomers help to further increase the rate of reaction and cure, enabling improved curing kinetics, as shown by DSC. The improved kinetics of the compositions of the present invention also widens the formulation window so that polymers from other addition polymerizable monomers, such as alkyl (meth)acrylates can be used.

The polymers of the present invention rely on orthogonal mechanisms of polymerization and ring opening curing. Ring opening curing in accordance with the present invention removes or reacts the arylcyclobutene or, preferably, BCB groups of the arylcyclobutene-containing monomers A by Diels-Alder ring opening. Independently, polymerizing or crosslinking comprises addition polymerization or curing or may comprise condensation reaction, such as esterification or amidation at a temperature well below 200 °C. As a result, cure temperatures and polymer use methods can to be tuned by selecting the curing or crosslinking mechanism of the polymer composition of the present invention depending on the intended application.

The present inventors have found that ring opening cure for aromatic group substituted addition polymerizable arylcyclobutene-containing monomers, such as vinyl phenoxy arylcyclobutenes, takes place at from 180 to 200 °C for 1 hour; further, ring opening cure for aliphatic group substituted addition polymerizable arylcyclobutenes as vinyl methyl arylcyclobutenes, takes place at from 200 to 220 °C for 1 hour.

In addition, because the polymer compositions in accordance with the present invention are addition polymerized, one can separately addition polymerize and cure the polymers by ring opening to enable tuning of the molecular weight of the resulting polymer according to the amount of arylcyclobutene group containing monomer A used.

Suitable addition polymerizable fourth monomers for useful in making the polymer compositions in accordance with the present invention are free of arylcyclobutene groups. Suitable fourth monomers can be, for example, (meth)acrylates, such as n-butyl (meth)acrylates and fatty (meth)acrylates, allyl group containing monomers, alkenes, dienes and addition polymerizable monomers containing additional functional groups, such as acidic or hydrolyzable groups, and high boiling point (normal boiling point of at least 150 °C) monomers, such as allyl, or maleimide terminated polyols, polyarylene ethers, or polysiloxanes.

The arylcyclobutene polymer compositions in accordance with the present invention may comprise as polymerized units one or more addition polymerizable fourth monomers comprising a diene, such as butadiene or 8-myrcene; or comprising an additional polymerizable group or a dienophile group, or two or more dienophile groups, such as diallyl ether, allyloxystyrene, or divinyl benzene. Fourth monomers comprising two or more addition polymerizable or dienophile groups may be used to impart addition crosslinking capabilities to the polymer composition of the present invention. Such monomers can also be used as a separate crosslinker in addition to the polymer. The selection of such fourth monomers is within the ability of those skilled in the art.

Suitable addition polymerizable fourth monomers that contain additional functional groups, such as acidic groups, for example, anhydride groups, or other hydrolyzable groups, such as active hydrogen or hydrolyzable sil(ox)ane groups, allow for modular post-polymerization functionalization or curing by condensation. One can, for example, react such monomers with a diamine or multifunctional amine to form an amic acid precursor which can be imidized during a thermal cure process. One can also hydrolyze the anhydride, such as by combining it with aqueous media and alcoholize such monomers, such as with t-butanol, to form monoesters.

In accordance with the methods of making the polymer composition of the present invention, the methods comprise polymerizing the monomer mixture of the present invention via non-controlled free radical addition polymerization using, for example benzoyl peroxide (BPO) or a persulfate or its salt as an initiator. In polymerizing, the monomer mixture is generally heated to from 60 to 140 °C, or, preferably, from 70 to 125 °C.

Suitable polymerization solvents are any organic solvents which dissolve the one or more monomers and have boiling points above the polymerization temperature of the monomers, such as mesitylene. Exemplary organic solvents include polar aprotic solvents such as amides, esters, such a butyl acetate, and sulfones, and polar protic solvents, such as (cyclo)alkanols.

Polymerization time is typically from 45 minutes to 60 hours, for example, 2 to 30 hours. For certain applications, it may be desired to stop the polymerization at the oligomer stage. Such oligomers composed of one or more monomers of the invention may be composed predominantly of dimers, trimers, tetramers, and the like, and may then be subsequently further polymerized. As used herein, the term "monomer(s) of the present invention" is intended to include the individual compounds described herein, as well as dimers, trimers and tetramers thereof which are then to be further polymerized.

In one example, the polymerizing of the monomer mixture in accordance with the present invention may be carried out at from 60 to 100 °C at a solids concentration of from 10 to 80 wt.%, or, preferably, from 20 to 50 wt.% of polymer solids using, for example, n-butyl acetate (bp = 126 °C) or 2-pentanone as solvent. After a period of time (4-24 hr), polymerizations are chased with additional BPO, then allowed to run for another 4-10 hr.

Prior to curing or crosslinking the polymer compositions in accordance with the present invention have a weight average molecular weight of from 3,000 to 250,000 or, preferably, from 6,000to 50,000, as determined by gel permeation chromatography (GPC) against polystyrene standards.

The polymer composition can be crosslinked via thermally induced or light induced initiation in the presence of the curing agent and a monomer containing two or more dienophile groups which are addition polymerizable, or by ring opening of the arylcyclobutene groups on the polymer in the presence of any monomer containing two or more dienophile groups.

The polymers of the present invention may be used as is or may be isolated by adding a non-solvent, such as water or methanol, to precipitate the polymer from the solution and thereafter removing the organic solvent.

The polymer compositions of the present invention, for example, thin film or coating compositions may comprise one or more arylcyclobutene-containing polymers and one or more organic solvents. Suitable organic solvents are those in which the polymers are soluble. Particularly useful organic solvents are any solvents useful in the making or formulation of arylcyclobutene polymers. Exemplary organic solvents include, without limitation: polar protic and polar aprotic solvents, for example: alcohols such as 2-methyl-1-butanol, 4-methyl-2-pentanol, and methyl isobutyl carbinol; esters such as ethyl lactate, propylene glycol methyl ether acetate, methyl 2-hydroxyisobutyrate, methyl 3-methoxypropionate, n-butyl acetate and 3-methoxy-1-butyl acetate; lactones such as gamma-butyrolactone; lactams such as N-methylpyrrolidinone; ethers such as propylene glycol methyl ether and dipropylene glycol dimethyl ether isomers, such as PROGLYDE™ DMM (The Dow Chemical Company, Midland, MI (Dow)); ketones such as 2-butanone, cyclopentanone, cyclohexanone and methylcyclohexanone; and mixtures thereof.

Suitable additives that may be useful in the polymer compositions of the present invention include, without limitation, one or more of each of curing agents, crosslinkers, such as crosslinking monomers separate from the polymer, surfactants, inorganic fillers, organic fillers, plasticizers, adhesion promoters, metal passivating materials, and combinations of any of the foregoing. Suitable surfactants are well- known to those skilled in the art, and nonionic surfactants are preferred. Such surfactants may be present in an amount of from 0 to 10 g/L, and preferably from 0 to 5 g/L. Any suitable inorganic fillers may optionally be used in the present compositions, and are well-known to those skilled in the art. Exemplary inorganic fillers include, but are not limited to, silica, silicon carbide, silicon nitride, alumina, aluminum carbide, aluminum nitride, zirconia, and the like, and mixtures thereof. The inorganic filler may be in the form of a powder, rods, spheres, or any other suitable shape. Such inorganic fillers may have any suitable dimensions. Such inorganic fillers may comprise a coupling agent, such as a silane or a titanate in conventional amounts.

Inorganic fillers may be used in an amount of from 0 to 80 wt.%, such as 40 to 80 wt.%, as solids based on the total weight of the composition.

Preferably, the metal passivating material is a copper passivating agent. Suitable copper passivating agents are well known in the art and include imidazoles, benzotriazoles, ethylene diamine or its salts or acid esters, and iminodiacetic acids or salts thereof.

Any condensation crosslinkers that react with any functional groups on any of the second monomers or any olefin group or dienophile group on the polymer of the present invention may be used as crosslinkers, provided that they crosslink with the arylcyclobutene polymer of the present invention under the conditions used to cure the composition. Suitable crosslinkers include, but are not limited to, diamines, polyamines, and polythiols, including polymers having multiple amine or thiol groups and added monomers having two or more olefin groups or dienophile groups, such as glycol di(meth)acrylates or diallyl phthalate and bis-arylcyclobutene monomers of formula (1) above wherein at least one of R¹ and R² is independently a monovalent hydrocarbon containing group, such as a C₁ to C₆ alkyl, carboxyalkyl, keto, aldehyde, acetal, ketal, or hydroxyalkyl group. The selection of such crosslinkers is within the ability of those skilled in the art. Such crosslinkers are typically used in an amount of from 0 to 20 wt.%, and preferably 0 to 10 wt.%, based on the total weight of the polymerizable monomers in the composition. The polymer compositions of the present invention find many uses, such as in photolithography, packaging, adhesive, sealing and bulk dielectric applications, such as in spin on coatings or buffer layers.

A variety of curing agents may be used in the polymer compositions of the present invention which are useful in photolithography. Suitable curing agents may aid in the curing of the bis-benzocyclobutene containing materials, and may be activated by heat or light. Exemplary curing agents include, but are not limited to, thermally generated initiators and photoactive compounds (photogenerated initiators). The selection of such curing agents is within the ability of those skilled in the art. Preferred thermal generated initiators are free radical initiators, such as, but not limited to, azobisisobutyronitrile, dibenzoyl peroxide, and dicumylperoxide. Preferred photoactive curing agents are free radical photoinitiators available from BASF under the Irgacure brand, and diazonaphthoquinone (DNQ) compounds including sulfonate esters of a DNQ compound. Suitable DNQ compounds are any compounds having a DNQ moiety, such as a DNQ sulfonate ester moiety, and that function as photoactive compounds in the present compositions, that is, they function as dissolution inhibitors upon exposure to appropriate radiation. Suitable DNQ compounds are disclosed in U.S. Pat. Nos. 7,198,878 and 8,143,360. The amount of photoactive compound varies from 0 to 30 wt.%, based on the total weight of the polymer solids. When present, the photoactive compound is typically used in an amount of 5 to 30 wt.%, preferably from 5 to 25 wt.%, and more preferably from 10 to 25 wt.%, based on the total weight of polymer solids.

Any suitable adhesion promoter may be used in the polymer compositions of the present invention and the selection of such adhesion promoter is well within the ability of those skilled in the art. Preferred adhesion promoters are silane-containing materials or tetraalkyl titanates, and more preferably trialkoxysilane-containing materials. Exemplary adhesion promoters include, but are not limited to: bis(trialkoxysilylalkyl)benzenes such as bis(trimethoxysilylethyl)benzene; aminoalkyl trialkoxy silanes such as aminopropyl trimethoxy silane, aminopropyl triethoxy silane, and phenyl aminopropyl triethoxy silane; and other silane coupling agents, as well as mixtures of the foregoing. Particularly suitable adhesion promoters include AP 3000, AP 8000, and AP 9000S, (Dow Electronic Materials, Marlborough, MA).

The polymer compositions of the present invention may contain from 0 to 15 wt.% of an adhesion promoter based on the total weight of the composition, preferably from 0.5 to 10 wt.%, more preferably from 1 to 10 wt.%, yet more preferably from 2 to 10 wt.%.

The photolithographic polymer compositions the present invention may be prepared by combining one or more polymers of the present invention and any organic solvents, water or additional components in any order. When the present compositions contain a curing agent such as a photoactive compound, such as a diazonaphthoquinone, an onium salt or photoinitiator, it is preferred that the curing agent is first dissolved in a suitable organic solvent or aqueous alkali, then combined with one or more present polymers and any optional surfactant, and then combined with any optional adhesion promoter. Selection of a suitable photoactive compound is within the ordinary level of skill in the art.

The bis-arylcyclobutene-containing polymer compositions of the present invention are useful in forming arylcyclobutene containing coatings that are more easily developable using aqueous alkali when compared to coatings prepared from conventional benzocyclobutene-containing polymers.

Any of the compositions of the present invention can be used to form a layer of a bis-arylcyclobutene suitable for use as dielectric layers, permanent bonding adhesives, as stress buffer layers, and the like.

The polymer compositions of the present invention may be coated on a substrate by any suitable method. Suitable methods for disposing the present compositions include, but are not limited to, spin-coating, curtain coating, spray coating, roller coating, dip coating, vapor deposition, and lamination such as vacuum lamination, among other methods. In the semiconductor manufacturing industry, spin-coating is a preferred method to take advantage of existing equipment and processes. In spin-coating, the solids content of the composition may be adjusted, along with the spin speed, to achieve a desired thickness of the composition on the surface it is applied to.

Typically, the polymer compositions of the present invention are spin-coated at a spin speed of 400 to 4000 rpm. The amount of the present compositions dispensed on the wafer or substrate depends on the total solids content in the composition, the desired thickness of the resulting layer, and other factors well-known to those skilled in the art. When a film or layer of the present compositions is cast, such as by spin-coating, much (or all) of the solvent evaporates during deposition of the film. Preferably, after being disposed on a surface, the composition is heated (baked) to remove any remaining solvent. Typical baking temperatures are from 90 to 180 °C, although other temperatures may be suitably used. Such baking to remove residual solvent is typically done for approximately 2 minutes, although longer or shorter times may suitably be used. The bis-arylcyclobutene polymers of the present invention are typically cured by heating for a period of time. Suitable curing temperatures range from ambient temperature to 210 °C. Typically curing times range from 1 to 600 minutes.

Preferably, layers of the polymer compositions of the present invention may also be formed as a dry film and disposed on the surface of a substrate by lamination. A variety of suitable lamination techniques, including vacuum lamination techniques, may be used and are well known to those skilled in the art. In forming a dry film, the present compositions are first disposed, such as coated, onto a front surface of a suitable film support sheet such as a polyester sheet, preferably polyethyleneterephthalate (PET) sheet, or a polyimide sheet such as KAPTON™ polyimide (DuPont, Wilmington, DE), using slot-die coating, gravure printing, or another appropriate method. The composition is then soft baked at a suitable temperature, such as from 90 to 140 °C, for an appropriate time, such as from 1 to 30 minutes, to remove any solvent. A polymer film cover sheet such as polyethylene is then roll-laminated at room temperature onto the dried composition to protect the composition during storage and handling. To dispose the dried composition onto the substrate, the cover sheet is first removed. Then, the dried composition on the support sheet is laminated onto the substrate surface using roll-lamination or vacuum lamination. The lamination temperature can range from 20 to 120 °C. The support sheet is then removed (peeled), leaving the dried composition on that surface. A wide variety of electronic device substrates may be employed in the present invention. An electronic device substrate is any substrate for use in the manufacture of any electronic device. Exemplary electronic device substrates include, without limitation, semiconductor wafers, glass, sapphire, silicate materials, silicon nitride materials, silicon carbide materials, display device substrates, epoxy mold compound wafers, circuit board substrates, and thermally stable polymers. As used herein, the term "semiconductor wafer" is intended to encompass a semiconductor substrate, a semiconductor device, and various packages for various levels of interconnection, including a single-chip wafer, multiple-chip wafer, packages for various levels, substrates for light emitting diodes (LEDs), or other assemblies requiring solder connections. Semiconductor wafers, such as silicon wafers, gallium-arsenide wafers, and silicon-germanium wafers, may be patterned or unpatterned. As used herein, the term "semiconductor substrate" includes any substrate having one or more semiconductor layers or structures which include active or operable portions of semiconductor devices. The term "semiconductor substrate" is defined to mean any construction comprising semiconductive material, such as a semiconductor device. A semiconductor device refers to a semiconductor substrate upon which at least one microelectronic device has been or is being fabricated. Thermally stable polymers include, without limitation, any polymer stable to the temperatures used to cure the arylcyclobutene material, such as polyimide, for example, KAPTON™ polyimide (DuPont, Wilmington, DE).

When compositions of the present invention which do not contain an adhesion promoter are used, the surface of the substrate to be coated with the present compositions may optionally first be contacted with a suitable adhesion promoter or vapor treated. Such treatments improve the adhesion of the bis-arylcyclobutene polymer compositions of the present invention to the substrate surface.

Suitable coating methods include any suitable method, such as spin-coating, dip coating, spray coating, curtain coating, roll coating, vapor deposition, and the like, may be used to contact the substrate surface with the adhesion promoter. Spin-coating is a preferred method for contacting the substrate surface with an adhesion promoter. Any suitable adhesion promoter may be used and the selection of such adhesion promoter is well within the ability of those skilled in the art. Preferred adhesion promoters are silane-containing materials, and more preferably trialkoxysilane-containing materials. Exemplary adhesion promoters useful to pretreat the substrate surface are those described above. Various vapor treatments known in the art may be used to increase the adhesion of the arylcyclobutene polymers of the present invention to the substrate surface, such as plasma treatments. In certain applications, it may be preferred to use an adhesion promoter o treat the substrate surface prior to coating the surface with the present compositions.

EXAMPLES: The present invention will now be described in detail in the following, non-limiting Examples:
Unless otherwise stated all temperatures are room temperature (21-23 °C) and all pressures are atmospheric pressure (-760 mm Hg or 101 kPa).

Notwithstanding other raw materials disclosed below, the following raw materials were used in the Examples:
A-POSS: multifunctional acrylate with an inorganic silsesquioxane at the core and organic acrylopropyl groups attached at the corners of the cage (CAS: 1620202-27-8, Hybrid Plastics);
BCB: benzocyclobutene;
BPO: Benzoyl peroxide;
BMI: Bismaleimide (unless otherwise indicated, FW 358.35);
BMI-TMH: bis-maleimide of 2,2,4-trimethyl hexane;
CL A: propoxylated trimethylolpropane triacrylate (SR492™, Sartomer, Exton, PA);
CL B: tricyclodecanedimethanol diacrylate (SR833s™, Sartomer);
DAAM: Diacetone acrylamide (2-Propenamide, N-(1,1-dimethyl-3-oxobutyl)-;
DVS or DVS-bis-BCB: (divinyl siloxane containing bisbenzocyclobutene monomer);
H126: Di-ally) bisphenol A, aka benzene, 1,1'-(1-methylethylidene)bis[4-(2-propen-1-yloxy)-] (CAS 3739-67-1);
Irganox™ 1010: Pentaerythritol tetrakis(3,5-di-tert-butyl-4-hydroxyhydrocinnamate) antioxidant (BASF, Ludwigshafen, DE);
MBA: 3-methoxy butylacetate solvent;
PET: poly(ethylene terephthalate);
MI: maleimide;
TAIC: triallyl isocyanurate, 1,3,5-Triazine-2,4,6(1H,3H,5H)-trione, 1,3,5-tri-2-propen-1-yl-(CAS 1025-15-6, Evonik, Essen, DE);
TMPTA: trimethylolpropane triacrylate (Sartomer. CAS 15625-89-5); and,
V601: A diazo radical initiator, dimethyl 2,2'-azobis(2-methylpropionate) (CAS No. 2589-57-3,Wako Chemical, Richmond, VA).

The materials were analyzed in various disclosed ways, including, as follows:
Mole Ratio Calculation: Moles of second, third and fourth monomers to moles of arylcyclobutene-containing monomer A was calculated from the starting number of moles of arylcyclobutene monomer. The residual exotherm from B-staging was measured in J/g and compared to the total enthalpy in the bis-arylcyclobutene monomer (in J/g, for DVS it is ∼800 J/g, corresponding to 156 J/mmole of BCB). The ratio of residual exotherm to enthalpy gave a number of mmoles of arylcyclobutene monomer per gram of solids; this was multiplied by the total mass in grams of solid, to give total mmoles of arylcyclobutene monomer. This was compared to number of mmoles of other monomers added to give the mole ratio.
Synthesis Example 1: Preparation of Low Curing BCB Polymer A: In this example, in a 100 mL EasyMaxTm reactor (Mettler, Toledo, Columbia, MD) with a polytetraflouroethylene (Teflon™ polymer, DuPont, Wilmington, DE) coated thermocouple and overhead mechanical stirring, was charged a heel of cyclopentanone (4.77 g) solvent and 4-vinyl pyridine (1.10 g). The reactor was cooled to 10 °C and purged with nitrogen for 20 minutes. In a separate 50 ml pear shaped round bottom flask, styrene (5.99 g), n-butyl acrylate (1.34 g), vinyl phenoxy BCB (6.11 g), and V601 (80 mg) were added, capped with a rubber septum and purged with nitrogen for 20 minutes. The resulting solution was drawn into a 50 mL syringe, and secured into a syringe pump. The reactor was heated to the reaction temperature (67 °C), and then the reaction solution was added via syringe pump at a rate of 4 mL/hr. After 5hr from the start of the addition, a chase of V601 initiator in 2-pentanone (40 mg in 4.83 g) was added via syringe at a right of 2 mL/hr. The reaction continued for a total of 20 hr then was cooled to room temperature and precipitated into methanol for use. GPC: Mn = 10.5k, Mw = 35.9k, PDI= 3.41.
Synthesis Example 2: Preparation of Low Curing BCB Polymer B: A polymer was made in the manner disclosed above for Synthesis Example 1, except that a heel of butyl acetate (13.0 g) solvent was charged. Further, to a separate 50 mL pear shaped round bottom flask were added, styrene (12.99 g), n-butyl acrylate (2.91 g), α-methyl vinyl BCB (11.44 g), and benzoyl peroxide (120 mg). The reactor was heated to a reaction temperature of 100 °C. After 24 hr from the start of the addition, a chase of benzoyl peroxide initiator in n-butyl acetate (40 mg in 7.71 g) was added via syringe at a right of 2 mL/hr. The polymer was poured into ajar for use. GPC: Mn = 29.9k, Mw = 109.4k, PDI= 3.65.
Polymer testing: The polymer of Example 1, the crosslinker and other materials, as indicated in Table 1, below, were combined and mixed in a 20 mL vial, draw coated on glass then peeled off the glass and placed into a DSC pan for analysis.

**Table 1: Curable Polymer Compositions**

| **Components** | **Ex 1** | **Ex 2** | **Ex 3** |
|---|---|---|---|
| Polymer A | 0.172 (g) | 0.155 (g) | |
| Polymer B | | | 0.725 (g) |
| Lrganox™^{,2} 1010 | 0.020 (g) | 0.020 (g) | |
| TAIC | 0.013 (g) | | 0.087 (g) |
| CL B | | 0.022 (g) | |

| | | | |
|---|---|---|---|
| 2. BASF. | | | |

The polymer curing kinetics were evaluated via differential scanning calorimetry (TA Instruments Q2000, TA Instruments, New Castle, DE), as disclosed below. Thermal stability was evaluated using thermogravimetric analysis (TA Instruments Q5000) under nitrogen atmosphere, as disclosed below. DSC and TGA information are presented in Table 2, below.

Differential Scanning Calorimetry (DSC): To give a glass transition temperature (Tg) DSC was performed with a TA Instruments Q2000 DSC device. A sample was weighed out (2-10 mg) and placed in an aluminum pan. A reference pan with no sample is placed in the reference slot of the device. The samples were equilibrated at 23 °C, then ramped linearly to 300 °C at a rate of 10 °C/minute under a nitrogen flow of 50 mL/min. The reported Tg is the inflection point of the resulting DSC curve.

Thermal stability was tested by running TGA analysis (Q500TM TGA instrument, 20 TA Instruments, New Castle, DE). A platinum 50 µL pan was tared then a polymer sample (1-10 mg) was loaded. The furnace is sealed and then the temperature is ramped from 25 °C to 600 °C at rate of 10 °C/min under nitrogen atmosphere 25 mL/min gas flow.

**Table 2: DSC and TGA Data**

| **Example** | **Peak Onset T (°C)** | **Peak Max T (°C)** | **Peak Energy (J/g)** |
|---|---|---|---|
| 1 | 150.74 | 188.95 | 189.6 |
| **2** | 150.27 | 181.62 | 213 |
| **3** | 197.53 | 230.87 | 307.7 |

As shown in Table 2, above, the onset cure temperature for a polymer of a phenoxy substituted arylcyclobutene-ring containing monomer A is much lower than that for polymer of an alkyl substituted arylcyclobutene-ring containing monomer A. As shown by Example 2, curing through DSC with an acrylate crosslinker CL B in Example 2 produces a sharper DSC peak than curing with a comparable allyl crosslinker, triallyl isocyanurate and may cure slightly faster; however, the allyl crosslinker enables easier orthogonal cure via addition and ring opening together.

Synthesis Example 3: Preparation of Polymer C (terpolymer 10%CHMA - 67% Sty - 23% a-methyl vinyl BCB): To a 100 mL EasyMax™ vessel equipped with septa, reflux condensor, nitrogen inlet, and overhead stirrer was added 13 g of n-butyl acetate. The solvent was flushed with nitrogen for 30 minutes, after which the solvent was heated to 100 °C. Concurrently, n-butylacetate (1.12 g), styrene (15.18 g, 0.146 mol), cyclohexylmethacrylate (3.66 g, 0.022 mol), alpha methyl-vinyl-benzocyclobutene (7.22 g, 0.05 mol) and benzoyl peroxide initiator (0.17g, 0.7 mmol, Sigma Aldrich, St. Louis, MO) were flushed with nitrogen for 30 minutes and added into a 100 mL Hamilton gastight syringe (Hamilton Company, Reno, NV). Once the internal temperature of the solvent had reached and stabilized at 108 °C, and the stir rate was set to 250 rpm the monomer feed was initiated at a rate of 20 mL per hour. At the completion of the addition, a small exotherm was observed. The reaction was allowed to stir for 24 hours, after which was added a nitrogen flushed solution of benzoyl peroxide in n-butyl acetate (0.06 g, 0.239 mmol) and n-butylacetate (7.39 g). This chase was added at a rate of 20 mL per hour and the reaction was allowed to continue for 10 hours. After this time, the polymer was cooled to room temperature and transferred into a pre-tarred glass bottle and kept at 55% solids. Polymer C was kept in solution. Polymer characterization included GPC (147.5k MW, 28.3k Mn, 5.2 PDI).

Comparative Example 3: Preparation of Comparative Polymer D (terpolymer 10%CHMA - 67% Sty - 23% Vinyl BCB): The polymer was synthesized as in Synthesis Example 3, above, but using vinyl BCB in place of α-methyl Vinyl BCB.

Solutions of the indicated polymer were cast onto a glass plate and drawn into films using a 76.2 micron (3 mil) steel draw down bar. Films were soft baked in a convection oven set at 120 °C for 10 min to remove solvent, and the polymer was collected by scraping the material off the plate using a razor blade. Cure data was collected using a Q2000 series DSC (TA Instruments). Cure point was determined by ramping ∼5 mg samples enclosed in an aluminum pan from 30 °C to 350 °C at a ramp rate of 10 °C/min, while cure amount or degree was determined by isothermally holding samples at the indicated temperature for the required amount of time before ramping the temperature from 30 °C to 350 °C to determine any residual unreacted BCB units. Note that "peak onset" is defined as the intersection of two tangent lines on a DSC curve. Data are presented in Table 3, below.

**Table 3: DSC Cure Onset**

| **Example** | **Polymer** | **Peak Onset** | **Peak Maximum** |
|---|---|---|---|
| 4 | C: α-MevBCB Sty polymer | 195 | 263 |
| 5* | D: vBCB Sty polymer | 215 | 269 |

| | | | |
|---|---|---|---|
| *- Denotes comparative Example | | | |

As shown in Table 3, above, the α-Me vinyl BCB-styrene Polymer C of Synthesis Example 3 reacts at lower temperatures than the comparative Example 3 styrene Polymer D comprising, in copolymerized form, a non-methylated vinyl BCB monomer. Although the α-Me vinyl BCB ring opens at a lower temperature, the curing end point (peak max) was similar to that of the comparative polymer D made using vinyl BCB because the polymers had a similar BCB level.

Additional data from the DSC analysis are presented in Table 4, below.

**Table 4: DSC Cure Timing**

| **Polymer** | **Cure T (°C)** | **Cure Time (hr)** | **Tg** (°C) | **Peak Temp (°C)** | **Area (J/g)** | **Conv %** |
|---|---|---|---|---|---|---|
| C | control | 0 | 68.8 | 265.7 | 203.5 | 0 |
| C | 180 | 1 | 115.8 | 269.2 | 142.6 | 29.9 |
| C | 180 | 2 | 121.7 | 270.5 | 137 | 32.7 |
| C | 200 | 1 | 138.1 | 274.9 | 90.9 | 55.3 |
| C | 200 | 2 | 147.4 | 277.2 | 80.2 | 60.6 |
| C | control | complete cure | 186.8 | | 0 | 100 |
| D* | control | 0 | 76.9 | 269.2 | 178.6 | 0 |
| D* | 180 | 1 | 104.3 | 274.4 | 164.6 | 7.8 |
| D* | 180 | 2 | 109.6 | 276.6 | 150.5 | 15.7 |
| D* | 200 | 1 | 119.3 | 281.1 | 128 | 28.3 |
| D* | 200 | 2 | 127 | 283.4 | 114 | 36.2 |
| D* | control | complete cure | 186 | | 0 | 100 |

| | | | | | | |
|---|---|---|---|---|---|---|
| *- Denotes comparative Example | | | | | | |

Polymer C of the present invention, made using α-Me vinyl BCB ring opens at a lower temperature than Comparative Polymer D, made using vinyl BCB (vBCB). Further, Polymer C, exhibited a dramatically increased conversion % when compared to the Comparative Polymer D made using a non-methylated vinyl BCB.

### Example 6: Cure Increase Using Different Crosslinkers

The formulations indicated in Table 5, below, were made by adding the indicated materials to a small cup and then blending for 30 seconds at 3500 rpm using a FlakTec™ speed mixer (FlackTek Inc, Landrum, SC), including a crosslinking monomer. Results are shown in Tables 6, 7, and 8, below.

**Table 5¹: Curable Formulations**

| **Example** | **6-1** | **6-2** | **6-3** |
|---|---|---|---|
| Materials | BMI-TMH | TAIC | SR492 |
| Polymer C | 0.37 | 0.44 | 0.37 |
| BMI-TMH | 0.071 | | |
| TAIC | | 0.043 | |
| CL A | | | 0.070 |
| Butyl Acetate | 0.056 | 0.018 | 0.055 |

| | | | |
|---|---|---|---|
| 1. Units are in grams. | | | |

**Table 6: DSC Cure of Materials**

| **Material** | **Onset T1** (°C) | **Peak Temp Max1 (°C)** | **Area1** (J/g) | **Onset T2** (°C) | **Peak Temp Max 2** (°C) | **Area 2** (J/g) |
|---|---|---|---|---|---|---|
| Polymer C+ CLA | 118.2 | 147 | 117.6 | 210.8 | 261 | 71.2 |
| CL A Only | 157 | 197 | 362.2 | N/A | N/A | N/A |
| Polymer C | 195.2 | 264.5 | 148 | N/A | N/A | N/A |
| Polymer C | 195.5 | 264.54 | 147 | | | -- |
| Polymer C +TAIC | 197.7 8 | 232.41 | 319.4 | -- | -- | -- |
| TAIC | 250.3 7 | 275.05 | 109.1 | -- | -- | -- |
| Polymer C | 195.5 | 264.54 | 147 | -- | -- | -- |
| Polymer C +BMI-TMH | 197.9 6 | 231.62 | 289.9 | -- | -- | -- |
| BMI-TMH | 238.3 6 | 264.49 | 73.24 | -- | -- | -- |

As shown in Table 6, above, the combination of crosslinker A and inventive Polymer C cured at a dramatically lower temperature than either the crosslinker or polymer alone. This indicates separate curing of each. However, the analysis in the case of TAIC or BMI-TMH indicates greater cure energy from their combination with the polymer, indicating ring opening cure.

In the following 2 tables, DSC analysis was modified as the temperature was held at the indicated cure temperature.

**Table 7: Isothermal Hold**

| **Material** | **Cure T (°C)** | **Cure Time (hr)** | **Onset T (°C)** | **Peak Temp Max (°C)** | **Area** (J/g) | **Conv, %** |
|---|---|---|---|---|---|---|
| Polymer C | polymer only | 0 | 68.8 | 265.7 | 203.5 | 0 |
| Polymer C + CLA | 180 | 2 | 219.3 | 268.74 | 84.85 | 58.3 |
| Polymer C + CLA | 200 | 2 | 237.59 | 275.81 | 46.82 | 77.0 |

As shown in Table 7, above, CL A does not incorporate into the polymer network by ring opening, and instead cures or converts to cured material separately. Addition crosslinking took place at below 200 °C.

**Table 8: DSC Cure Timing**

| **Material** | **Cure T (°C)** | **Cure Time** (hr) | **Tg (°C)** | **Peak Temp (°C)** | **Area** (J/g) | **Conv %** |
|---|---|---|---|---|---|---|
| Polymer C | control | 0 | 68.8 | 265.7 | 203.5 | 0 |
| Polymer C | 180 | 2 | 121.7 | 270.5 | 137 | 32.7 |
| Polymer C | 200 | 2 | 147.4 | 277.2 | 80.2 | 60.6 |
| Polymer C +TAIC | control | 0 | 42.2 | 232 | 335.3 | 0 |
| Polymer C+TAIC | 180 | 2 | 142.5 | 234.4 | 63.5 | 81.1 |
| Polymer C+BMI-TMH | control | 0 | N/A | 231.7 | 307.4 | 0 |
| Polymer C+TAIC | 200 | 2 | 181.3 | 264.9 | 1.8 | 99.5 |
| | | | | | | |
| Polymer C+BMI-TMH | 180 | 2 | 126.3 | 233.8 | 65.1 | 78.8 |
| Polymer C+BMI-TMH | 200 | 2 | 161.9 | 264.5 | 3.5 | 98.9 |

| | | | | | | |
|---|---|---|---|---|---|---|
| "- Denotes comparative Example | | | | | | |

The area under the largest peaks as well as the temperature corresponding to the peak and then diminishing area in the above Table shows that the TAIC or the BMI-TMH crosslinker react with polymer in a single ring opening, addition and overall complete cure at a significantly reduced cure temperature. The above table indicates complete cure within 2 hours as shown by Conv%.

## Claims

1. A polymer composition comprising, in copolymerized form, a monomer mixture of one or more addition polymerizable arylcyclobutene-containing monomers A R₁ having as a cyclobutene ring substituent one or more groups chosen from alkyl; heteroatom containing alkyl; aryl; heteroatom containing aryl; or heteroatom containing aryloxy, one or more aromatic addition polymerizable second monomers, and one or more other addition polymerizable monomers chosen R2 from an addition polymerizable nitrogen heterocycle containing third monomer, an addition R3 polymerizable fourth monomer, or both of the one or more third monomers and the one or more fourth monomers.

2. The polymer composition as claimed in claim 1, wherein the polymer composition comprises the at least one polymer of, in copolymerized form, a monomer mixture of one or more addition polymerizable arylcyclobutene-containing monomers A having as a cyclobutene ring substituent one or more groups chosen from alkyl and aryl and the polymer compositions further comprise an addition polymerizable crosslinker monomer containing two or more allyl groups.

3. The polymer composition as claimed in claim 1, wherein the polymer composition comprises, in copolymerized form, the monomer mixture wherein the one or more arylcyclobutene-containing monomers A has Structure A-1 or a Structure A-2: 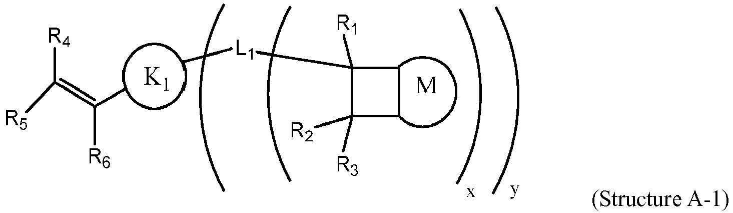 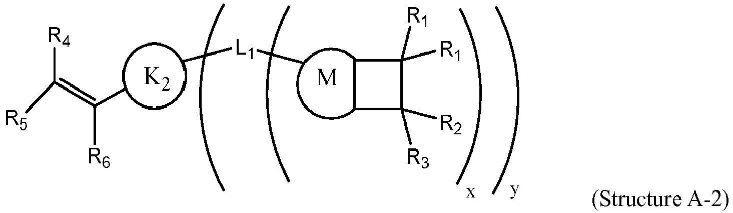
wherein, K₁ is a divalent heteroatom containing hydrocarbon group having from 1 to 36 carbon atoms; a divalent hydrocarbon group having from 1 to 36 carbon atoms; a cyano group; a carbonyl group; an ester group (-COO-); a carboxyl group (-OOC-); or is a divalent heteroatom group;
K₂ is a covalent bond or a divalent group chosen from a C₁ to C₆ alkyl substituted or unsubstituted divalent aryl group; a C₁-C₆ alkyl substituted or unsubstituted divalent hydrocarbon group; a divalent C₁ to C₃₀ alkylene group; a divalent heteroatom containing hydrocarbon group; an ether group (-O-); a thioether group (-S), a carbonyl group; an ester group (-COO-); a carboxyl group (-OOC-) or a cyano group;
M is a divalent aromatic group chosen from a C₁ to C₆ alkyl substituted or unsubstituted aromatic radical group, or a Ci to C6 alkyl substituted or unsubstituted divalent heteroaromatic radical group;
L₁ is a covalent bond or is a hydrocarbon linking group having a valence of x +1;
R₁ through R₆ are each independently selected from a monovalent group chosen from hydrogen, deuterium, halogen, hydroxyl a C₁ to C₆ alkyl group, a C₁ to C₆ alkoxy group, a C₁ to C₆ alkyl substituted hydrocarbon group, a heteroatom containing hydrocarbon group, a C₁ to C₆ alkyl substituted heterohydrocarbon group, a cyano group, an hydroxyl group, a monovalent aryl group, a C₁ to C₆ alkyl substituted aryl group, a heteroaryl group, or a C₁ to C₆ alkyl substituted heteroaryl group; and,
x and y are each independently an integer from 1 to 5 wherein y is 1 when L₁ is a covalent bond; wherein, when the one or more arylcyclobutene-containing monomers A has the structure A-1, each of R₁, R₂ and R₃ is a hydrogen; and,
further wherein, when the one or more arylcyclobutene-containing monomers A has the structure A-2; and at least one of R₁, R₂ and R₃ is chosen from a C₁ to C₆ alkyl group; a C₁ to C₆ alkoxy group; a C₁ to C₆ alkyl substituted hydrocarbon group having from 5 to 36 carbon atoms; a heteroatom containing hydrocarbon group; a C₁ to C₆ alkyl substituted heterohydrocarbon group; a cyano group; an aryl group; a C₁ to C₆ alkyl substituted aryl group; a heteroaryl group; or a C₁ to C₆ alkyl substituted heteroaryl group.

4. The polymer composition as claimed in claim 3, wherein the one or more arylcyclobutene-containing monomers A having the Structure A-1, wherein K is a an ether group, a thioether group, or a C₁ to C₆ alkyl substituted or unsubstituted divalent 0-heteroaryl group or S-heteroaryl group having from 5 to 36 carbons.

5. The polymer composition as claimed in claim 3, wherein in the one or arylcyclobutene-containing monomers A, x and y are each independently an integer of from 1 to 2.

6. The polymer composition as claimed in claim 3, wherein the one or more arylcyclobutene-containing monomers A comprise an α-vinyl phenoxy benzocyclobutene, α-vinyl methoxy benzocyclobutene, α-vinyl phenyl BCB or an α-vinyl hydroxynaphthalene BCB monomer of structure A-1, a vinyl α-alkoxy benzocyclobutene, a vinyl α-phenoxy benzocyclobutene, or a vinyl α-C₁ to C₆ alkyl benzocyclobutene monomer of structure A-2.

7. The polymer composition as claimed in claim 1, comprising a polymer of, in copolymerized form, a monomer mixture of the one or more addition polymerizable arylcyclobutene-containing monomers A, the one or more aromatic addition polymerizable second monomers chosen from styrene, α-methyl styrene, 6-myrcene, allyloxystyrene, allyl terminated polyarylene ethers or maleimide terminated polyarylene ethers; and the nitrogen heterocycle containing addition polymerizable third monomer.

8. The polymer composition as claimed in claim 1, comprising a polymer of, in copolymerized form, a monomer mixture of the one or more addition polymerizable arylcyclobutene-containing monomers A; the one or more aromatic addition polymerizable second monomers; and the one or more nitrogen heterocycle containing addition polymerizable third monomer chosen from N-vinyl pyridine, N-vinyl imidazole or other vinyl pyridine isomers or two or more addition polymerizable group containing nitrogen heterocycle containing third monomers.

9. The polymer composition as claimed in claim 1, comprising a polymer of, in copolymerized form, a monomer mixture od from 10 to 90 wt.% of the one or more arylcyclobutene-containing monomers A; from 5 to 70 wt.% of the one or more aromatic addition polymerizable second monomers; from 5 to 40 wt.% of the one or more other addition polymerizable monomers which is the one or more nitrogen heterocycle containing addition polymerizable third monomers, the one or more addition polymerizable fourth monomers, or both the third monomer and the fourth monomer, all weights based on the total solids weight of monomers used to make the copolymer with all monomer wt.%s adding to 100%.

10. The polymer composition as claimed in claim 1, comprising a polymer of, in copolymerized form, a monomer mixture of the one or more addition polymerizable arylcyclobutene-containing monomers A, the one or more aromatic addition polymerizable second monomers, and the one or more other addition polymerizable monomers which is one or more addition polymerizable fourth monomers chosen from acrylates or methacrylates; maleimides and bis-maleimides; cyclic anhydrides; (meth)acrylates for improving adhesion promotion; allyl group containing monomers for adhesion promotion; linear and branched alkenes; cyclic olefins; and fourth monomers containing a second dienophile or addition polymerizable group.

11. A method of making thin films or coatings on a substrate comprise depositing and evaporating, or coating on a substrate the polymer composition as claimed in claim 1.
